# EUROPEAN PATENT APPLICATION

(11) **EP 4 299 008 A1**
(43) Date of publication of application: **03.01.2024**
(21) Application number: 22759196.3
(22) Date of filing: 25.01.2022
(51) Int. Cl.: A61B 6/00, G16H 30/00, A61B 5/00

(54) **ASSESSMENT SYSTEM, ASSESSMENT SYSTEM CONTROL METHOD, AND CONTROL PROGRAM**

(30) Priority: 26.02.2021 JP 2021030858
(71) Applicant: Kyocera Corporation, Kyoto-shi Kyoto 612-8501 (JP)
(72) Inventor: WATANABE, Kenichi, Kyoto-shi, Kyoto 612-8501 (JP); KYOMOTO, Masayuki, Kyoto-shi, Kyoto 612-8501 (JP); HASHIDA, Masahiko, Kyoto-shi, Kyoto 612-8501 (JP); HONDA, Shintaro, Kyoto-shi, Kyoto 612-8501 (JP); WADA, Naoya, Kyoto-shi, Kyoto 612-8501 (JP)
(74) Representative: TBK
(86) International application number: PCT/JP2022/002489
(87) International publication number: WO 2022/181154

(57) **Abstract**

A user is presented with determination information obtained by determining a condition of a target site and feature information indicating a feature related to a condition of a body of a subject, which are generated from the same image representing the target site of the subject. A determination system includes an acquirer that acquires a target image representing a target site of a body of a subject, a first generator that generates first determination information indicating a condition of the target site from the target image used to generate the first determination information, a second generator that generates feature information indicating a feature related to a condition of the body of the subject from the target image, and a display controller that causes a display apparatus to display the first determination information and the feature information.

## Description

### TECHNICAL FIELD

The present disclosure relates to a determination system that presents determination results related to the present or future condition of a target site of a human body, a method of controlling the determination system, and a control program.

### BACKGROUND OF INVENTION

As described in Patent Document 1, technology for causing artificial intelligence (AI) to determine the present and future matters has been contrived.

### CITATION LIST

### PATENT LITERATURE

Patent Document 1: JP 2008-36068 A

### NON-PATENT LITERATURE

Non-Patent Document 1: Selvaraju et al., "Grad-CAM: Visual Explanations from Deep Networks via Gradient-based Localization", arXiv:1610.02391v4[cs.CV], 2019.

### SUMMARY

In an aspect of the present disclosure, a determination system includes an acquirer that acquires a target image representing a target site of a body of a subject; a first generator that generates first determination information indicating a condition of the target site from the target image; a second generator that generates feature information indicating features related to a condition of the body of the subject from the target image used to generate the first determination information; and a display controller that acquires the first determination information and the feature information and causes a display apparatus to display the first determination information and the feature information.

In an aspect of the present disclosure, a method of controlling a determination system includes acquiring a target image representing a target site of a body of a subject; generating first determination information obtained by determining a condition of the target site from the target image; generating feature information indicating features related to a condition of the body of the subject from the target image used to generate the first determination information; and acquiring the first determination information and the feature information and causing a display apparatus to display the first determination information and the feature information.

In each of the aspects of the present disclosure, the determination system may be implemented by a computer, and in this case, a control program of the determination system that causes the computer to implement the determination system by causing the computer to operate as each part (software element) included in an apparatus included in the determination system and also a computer-readable recording medium recording the control program are within the scope of the present disclosure.

### BRIEF DESCRIPTION OF THE DRAWINGS

FIG. 1 is a block diagram illustrating an example of a configuration of a determination system according to an aspect of the present disclosure.
FIG. 2 is a functional block diagram illustrating an example of a configuration of the determination system.
FIG. 3 is a diagram illustrating an example of a data structure of a display information database.
FIG. 4 is a diagram illustrating an example of first determination information generated from a target image representing lungs of a subject.
FIG. 5 is a diagram illustrating an example of first determination information generated from a target image representing a knee joint of a subject.
FIG. 6 is a flowchart illustrating an example of a flow of processing executed by an information processing apparatus of the determination system.
FIG. 7 is a diagram illustrating an example of a display screen.
FIG. 8 is a diagram illustrating an example of a display screen.
FIG. 9 is a diagram illustrating an example of a display screen.
FIG. 10 is a diagram illustrating an example of a display screen.
FIG. 11 is a diagram illustrating an example of a display screen.
FIG. 12 is a diagram illustrating an example of a display screen.
FIG. 13 is a diagram illustrating an example of a display screen.
FIG. 14 is a diagram illustrating an example of a display screen.
FIG. 15 is a flowchart illustrating another example of a flow of processing executed by the determination system.
FIG. 16 is a functional block diagram illustrating an example of a configuration of a determination system.
FIG. 17 is a diagram illustrating an example of a data structure of a display information database.
FIG. 18 is a functional block diagram illustrating an example of a configuration of a determination system.
FIG. 19 is a functional block diagram illustrating an example of a configuration of a determination system.
FIG. 20 is a diagram illustrating an example of a data structure of a display information database.
FIG. 21 is a diagram illustrating an example of a display screen.
FIG. 22 is a diagram illustrating an example of a display screen.
FIG. 23 is a functional block diagram illustrating an example of a configuration of a determination system.
FIG. 24 is a block diagram illustrating an example of a configuration of the determination system according to an aspect of the present disclosure.

### DESCRIPTION OF EMBODIMENTS

### First Embodiment

An embodiment of the present disclosure will be described in detail below.

### Overview of Determination System 100

In an aspect of the present disclosure, a determination system 100 acquires a target image representing a target site of a body of a subject and generates first determination information indicating a condition of the target site from the target image. The determination system 100 generates feature information indicating a feature related to a condition of the body of the subject from the target image used for generation of the first determination information.

The determination system 100 displays the first determination information and the feature information on a display apparatus 5.

The determination system 100 can present a user with the first determination information and the feature information, which are generated from the same target image representing the target site of the body of the subject. The feature information is information useful for the user to judge reliability of the first determination information. With reference to the feature information, the user can understand a reason why the first determination information is generated from the target image and can judge validity and reliability of the first determination information.

In this description, the target site may be any site of the body of the subject, and may be, for example, any one of a whole body, a head, a neck, an arm, a trunk, a waist, buttocks, a leg, a foot, and the like.

The target image may be a medical image representing the target site of the subject. In an example, the target image may be a medical image representing the target site of the subject, in order to examine the subject. Here, the medical image may be any one of an X-ray image, a computed tomography (CT) image, a magnetic resonance imaging (MRI) image, a positron emission tomography (PET) image, a radio isotope (RI) image, a mammographic image, an ultrasonic image, an endoscopic image, and an angiographic image, each representing the subject. The target image may be an image representing any one of a bone, a joint, a muscle, fat (subcutaneous fat and visceral fat), an internal organ, a blood vessel, and the like of the target site.

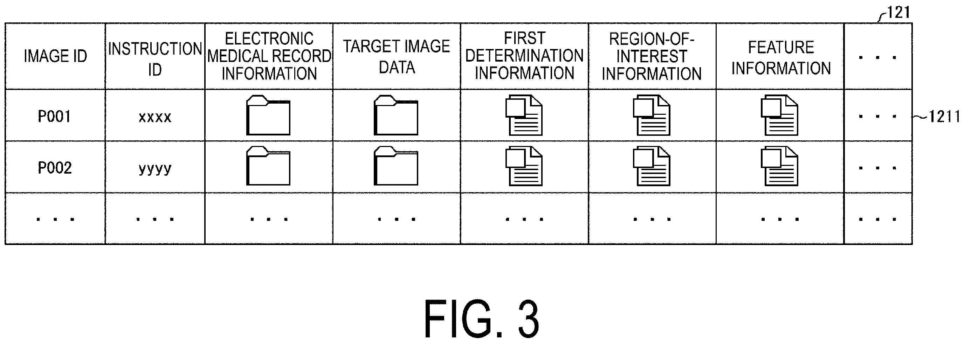

The first determination information may be information used to determine the condition of the target site at a first time when the target image is captured. Alternatively, the first determination information may include information used to determine the condition of the target site at a second time after the elapse of a predetermined period since the first time when the target image is captured (see FIG. 3).

Here, the first time may be a time when the medical image representing the target site of the subject is acquired, for example. Typically, the first time may be a time when a subject image representing the present condition of the target site of the subject is acquired. That is, the first time may be intended to be substantially the present time. The predetermined period may be any period that has elapsed since the first time, and may be half a year, may be a year, may be five years, may be ten years, or may be fifty years. That is, the second time may be intended to be substantially any time in future. The predetermined period is not limited to one period and may include a plurality of periods. That is, the first determination information may be information indicating determination results obtained by artificial intelligence (AI, for example, a first determination information generation apparatus 2 to be described below) and the like included in the determination system 100 determining the present or future condition of the target site of the subject, based on the target image.

The first determination information may be diagnostic information including diagnostic results related to whether the subject has a disorder. The diagnostic information may include presence or absence of a disorder, and a degree of progress or a degree of severity of the disorder. The diagnostic information may include a numerical value used to determine whether a disorder is present. The diagnostic information may include risks of a future disorder and risks of future symptoms such as a pain. When the disorder is not present, the diagnostic results may be those indicating "within normal limits" or "no problem". Information indicating a presymptomatic disease condition, such as information "the diagnostic results are within normal limits but a slight sign of a disorder can be observed", may be included. Information indicating "detailed examination required" and information indicating "determination impossible" may be included.

The feature information may be information indicating a feature detected from the target image by artificial intelligence (AI, for example, a feature information generation apparatus 3 to be described below) and the like included in the determination system 100. The feature information may include identification information including a name of each feature detected from the target image, and position information indicating a position of each feature in the target image. The feature information may be information indicating presence of any abnormality (symptom) detected in the target image and may be the position information indicating a position at which the abnormality is detected, when an image representing a normal target site and the target image are compared. The feature information may include a feature irrelevant to the target site of the subject. The feature information may include a feature irrelevant to determination of the condition of the target site.

The feature may be any feature in an image obtained by analyzing the target image. In an example, the feature may be at least one selected from the group consisting of an image of a malignant tumor, an image of a benign tumor, an image of a thrombus, an image of angiosclerosis, ground glass opacity, calcified focus opacity, a crazy-paving pattern, a patchy shadow, an image of interstitial change, an image of a pulmonary cyst, an image of pulmonary emphysema, an image of vascular compression, an image of nerve compression, an image of intervertebral disc degeneration, an image of a meniscus injury, an image of a ligament injury, an image of osteosclerosis, an image of a bone fracture, an image of a bone deformity, an image of development of an osteophyte, and an image of joint space narrowing.

The determination system 100 may further generate region-of-interest information indicating a region of interest related to the first determination information, which is a partial region of the target image. In this case, the determination system 100 displays, on the display apparatus 5, the first determination information, and the feature information and a region-of-interest image generated from the target image used for generation of the first determination information.

The region-of-interest information may be information indicating a position of the region of interest, which is a region of interest for the first determination information generation apparatus 2 in a process of generating the first determination information. That is, the region of interest may be a region of interest in the process in which the first determination information is generated from the target image, and the region-of-interest information may be information indicating the position of the region of interest in the target image.

For example, when the target image is an X-ray image of lungs (target site) and the diagnostic information is a disorder of pneumonia, the region-of-interest information may be information indicating an area around ground glass opacity. For example, when the target image is an X-ray image of a knee (target site) and the diagnostic information is a disorder of knee osteoarthritis, the region-of-interest information may be information indicating an area around a part corresponding to joint space narrowing, a part corresponding to osteosclerosis, or the like.

The determination system 100 displays, on the display apparatus 5, the region-of-interest information in addition to the first determination information and the feature information and can thereby more effectively enhance understanding of the user regarding the first determination information. In this description, description will be given by taking an example of a case in which the determination system 100 can generate the first determination information, the feature information, and the region-of-interest image. However, a configuration of also generating the region-of-interest image in addition to the first determination information and the feature information is not essential.

### Schematic Configuration of Determination System 100

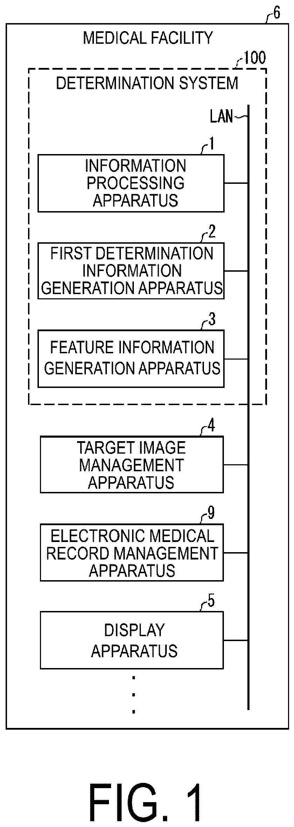

The following will describe a configuration of the determination system 100 by taking an example of a medical facility 6 that employs the determination system 100. FIG. 1 is a block diagram illustrating a configuration example of the determination system 100. In this case, the subject may be a patient who undergoes an examination and treatment related to a condition of a target site in the medical facility 6. The target image may be a medical image representing the target site of the subject. The following description will be given by taking an example of a case in which the target site is lungs and a knee of the subject; however, the target site is not limited to these.

As illustrated in FIG. 1, the determination system 100 may include an information processing apparatus 1, a first determination information generation apparatus 2, and a feature information generation apparatus 3. Each of the information processing apparatus 1, the first determination information generation apparatus 2, and the feature information generation apparatus 3 is a computer. The first determination information generation apparatus 2 and the feature information generation apparatus 3 are communicably connected to the information processing apparatus 1. As illustrated in FIG. 1, the information processing apparatus 1, the first determination information generation apparatus 2, and the feature information generation apparatus 3 may be connected to a local area network (LAN) of the medical facility 6.

The medical facility 6 may include a target image management apparatus 4 and one or more display apparatuses 5, in addition to the determination system 100. As illustrated in FIG. 1, both of the target image management apparatus 4 and the display apparatus(es) 5 may be connected to the LAN of the medical facility 6.

The first determination information generation apparatus 2 is a computer that can generate the first determination information from the target image. The first determination information generation apparatus 2 may be able to generate the region-of-interest information from the target image. The first determination information generation apparatus 2 will be described below.

The feature information generation apparatus 3 is a computer that can generate the feature information from the target image. The feature information generation apparatus 3 will be described below.

The information processing apparatus 1 acquires the target image from the target image management apparatus 4. The information processing apparatus 1 transmits the target image to the first determination information generation apparatus 2 and instructs the first determination information generation apparatus 2 to generate the first determination information and the region-of-interest information. The information processing apparatus 1 transmits the same target image to the feature information generation apparatus 3 and instructs the feature information generation apparatus 3 to generate the feature information. The information processing apparatus 1 receives the first determination information and the region-of-interest information from the first determination information generation apparatus 2 and receives the feature information from the feature information generation apparatus 3.

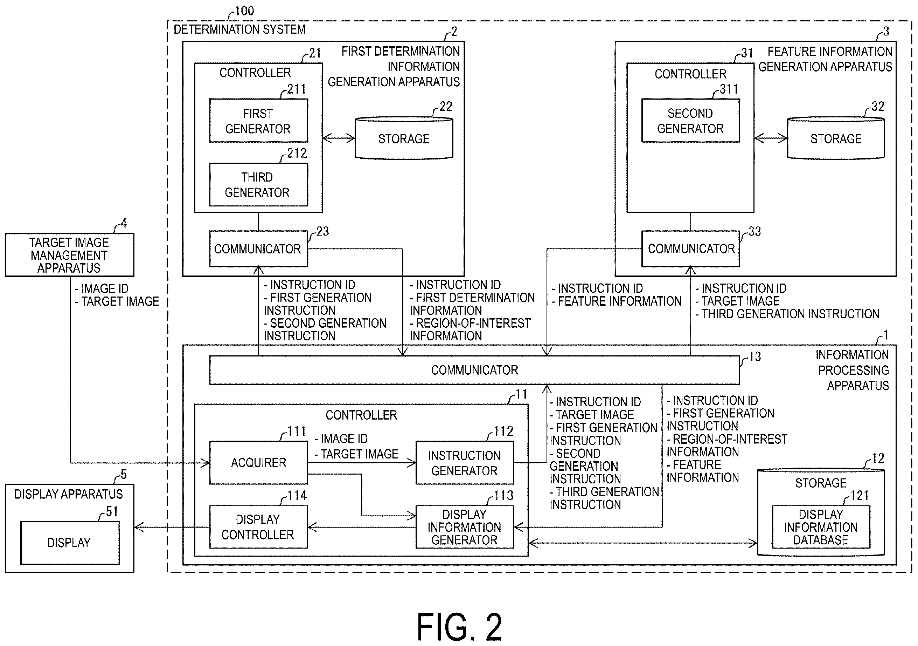

The display apparatus 5 includes a display 51 (for example, see FIG. 2) that can receive the first determination information, the region-of-interest information, and the feature information from the information processing apparatus 1 and display these pieces of information. The display apparatus 5 may be a computer used by medical staff, such as medical doctors, who belong to the medical facility 6.

The display apparatus 5 may be, for example, a personal computer, a tablet terminal, a smartphone, or the like, and may include a communicator for performing transmission and reception of data to and from another apparatus and an input unit such as a keyboard and a microphone. In this case, the display apparatus 5 can receive various instruction inputs from the medical staff.

In the determination system 100 illustrated in FIG. 1, the first determination information generation apparatus 2 generates the first determination information and the region-of-interest information, but this is not restrictive. For example, the first determination information generation apparatus 2 may generate only the first determination information, and another apparatus (not illustrated) different from the first determination information generation apparatus 2 may generate the region-of-interest information. In this case, the information processing apparatus 1 transmits the target image to such another apparatus and instructs such another apparatus to generate the region-of-interest information.

The target image management apparatus 4 is a computer that functions as a server for managing the target image. In an example, in response to an instruction from the medical staff input to the display apparatus 5, the target image management apparatus 4 may transmit the target image designated in the instruction to the information processing apparatus 1. In this case, the instruction from the medical staff may include identification information specific to each subject (a subject ID to be described below), identification information specific to each target image (an image ID to be described below), and a MAC address of the information processing apparatus 1.

An electronic medical record management apparatus 9 is a computer that functions as a server for managing electronic medical record information of a subject who has undergone an examination in the medical facility 6. In this case, each apparatus of the determination system 100 may acquire basic information related to the subject from the electronic medical record management apparatus 9. Here, the basic information is information included in the electronic medical record information of the subject and may include at least one selected from the group consisting of pieces of information indicating gender, age, height, and weight of the subject, and the present health condition of the subject (at the first time). The basic information may include a subject ID being identification information specific to each subject, which is assigned to each subject.

FIG. 1 illustrates an example in which the information processing apparatus 1, the first determination information generation apparatus 2, the feature information generation apparatus 3, the target image management apparatus 4, and the electronic medical record management apparatus 9 are connected to the LAN disposed in the medical facility 6, but this is not restrictive. For example, as the network in the medical facility 6, the Internet, a telephone communication line network, an optical fiber communication network, a cable communication network, a satellite communication network, or the like may be applied. The LAN in the medical facility 6 may be communicably connected to an external communication network.

In the determination system 100, the information processing apparatus 1 and at least one selected from the group consisting of the display apparatus 5, the first determination information generation apparatus 2, the feature information generation apparatus 3, the target image management apparatus 4, and the electronic medical record management apparatus 9 may be directly connected to each other without use of the LAN. A plurality of display apparatuses 5, first determination information generation apparatuses 2, feature information generation apparatuses 3, target image management apparatuses 4, and electronic medical record management apparatuses 9 that can communicate with the information processing apparatus 1 may be provided. In the determination system 100, a plurality of information processing apparatuses 1 may be introduced.

### Configuration of Determination System 100

Configurations of the information processing apparatus 1, the first determination information generation apparatus 2, and the feature information generation apparatus 3 of the determination system 100 will be described with reference to FIG. 2. FIG. 2 is a functional block diagram illustrating an example of a configuration of the determination system 100. FIG. 2 also illustrates the target image management apparatus 4 and the display apparatus 5, which are communicably connected to the determination system 100.

The following will describe the information processing apparatus 1, the first determination information generation apparatus 2, and the feature information generation apparatus 3.

### Information Processing Apparatus 1

The information processing apparatus 1 includes a controller 11 that comprehensively controls each part of the information processing apparatus 1, a storage 12 that stores various pieces of data used by the controller 11, and a communicator 13 that performs communication of various pieces of data.

The controller 11 includes an acquirer 111, an instruction generator 112, a display information generator 113, and a display controller 114.

As illustrated in FIG. 2, the acquirer 111 acquires the target image from the target image management apparatus 4. Alternatively, the acquirer 111 may acquire the target image from a computer (for example, the display apparatus 5) used by a medical doctor. Together with the target image, the acquirer 111 may acquire an image ID being identification information specific to each target image, which is assigned to each target image.

The acquirer 111 may acquire one or more target images respectively corresponding to a plurality of subjects. When acquiring target images respectively corresponding to a plurality of subjects, the acquirer 111 may acquire subject IDs in addition to the image IDs.

The instruction generator 112 acquires the target images and the image IDs and generates various instructions. The instruction generator 112 generates a first generation instruction and a second generation instruction to be transmitted to the first determination information generation apparatus 2, and a third generation instruction to be transmitted to the feature information generation apparatus 3. The first generation instruction is an instruction for causing the first determination information generation apparatus 2 to generate the first determination information from the target image, and the second generation instruction is an instruction for causing the first determination information generation apparatus 2 to generate the region-of-interest information from the target image. The third generation instruction is an instruction for causing the feature information generation apparatus 3 to generate the feature information from the target image.

The first generation instruction and the second generation instruction may include a MAC address of the first determination information generation apparatus 2 being a transmission destination and a MAC address of the information processing apparatus 1 being a transmission source. On the other hand, the third generation instruction may include a MAC address of the feature information generation apparatus 3 being a transmission destination and a MAC address of the information processing apparatus 1 being a transmission source.

In an example, the instruction generator 112 may assign a specific instruction ID to each of the generated instructions. In this case, each of the first generation instruction, the second generation instruction, and the third generation instruction that are transmitted together with the target image assigned the same image ID may be assigned instruction IDs associated with each other.

The first generation instruction and the second generation instruction generated by the instruction generator 112 are transmitted to the first determination information generation apparatus 2 via the communicator 13, together with the instruction ID and the target image. On the other hand, the third generation instruction generated by the instruction generator 112 is transmitted to the feature information generation apparatus 3 via the communicator 13, together with the instruction ID and the target image.

The display information generator 113 acquires, for each target image, (1) the first determination information and the region-of-interest information generated by the first determination information generation apparatus 2 and (2) the feature information generated by the feature information generation apparatus 3. The display information generator 113 refers to the instruction ID and the like associated with each piece of acquired information, associates the first determination information and the region-of-interest information and the feature information, and generates display information. The display information generator 113 may store the generated display information in a display information database 121 of the storage 12, together with the instruction ID.

### Display Information Database 121

Here, the display information database 121 will be described with reference to FIG. 3. FIG. 3 is a diagram illustrating an example of a data structure of the display information database 121.

The display information database 121 records the following pieces of information, which are associated with each other.
- The instruction ID.
- The first determination information and the region-of-interest information generated by the first determination information generation apparatus 2 in response to the first generation instruction and the second generation instruction assigned the instruction ID.
- The feature information generated by the feature information generation apparatus 3 in response to the third generation instruction assigned the instruction ID.

The display information database 121 may further record the following pieces of information, which are associated with each other.
- The image ID of the target image transmitted to the first determination information generation apparatus 2 and the feature information generation apparatus 3 together with the first generation instruction, the second generation instruction, and the third generation instruction assigned the instruction ID.
- The electronic medical record information of the subject (which may include the subject ID) shown on the target image assigned the image ID.

Referring back to FIG. 2, the display controller 114 causes the display 51 of the display apparatus 5 to perform display, using each piece of information associated for each instruction ID from the display information database 121.

### First Determination Information Generation Apparatus 2

The first determination information generation apparatus 2 includes a controller 21 that comprehensively controls each part of the first determination information generation apparatus 2, a storage 22 that stores various pieces of data used by the controller 21, and a communicator 23 that performs communication of various pieces of data.

The controller 21 includes a first generator 211 that generates the first determination information and a third generator 212 that generates the region-of-interest information, based on the target image.

### First Generator 211

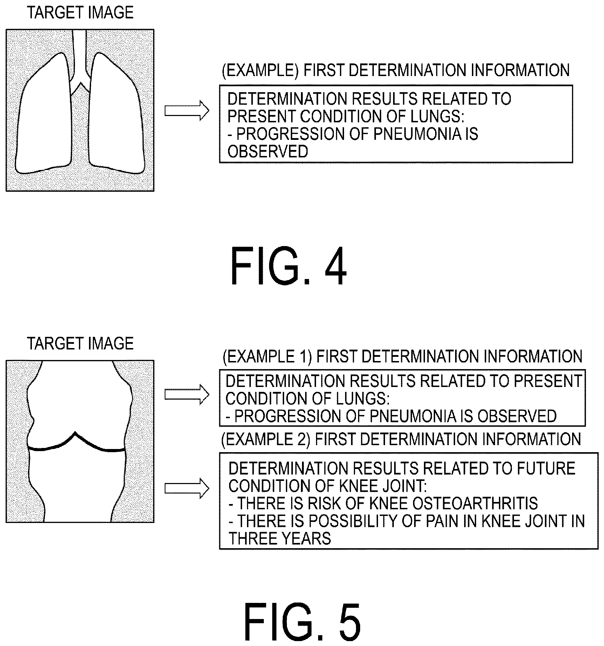

The first generator 211 generates, from the target image, the first determination information related to the target site at the first time or at the second time after the elapse of the predetermined period since the first time. FIG. 4 is a diagram illustrating an example of the first determination information generated from the target image representing lungs of the subject. FIG. 4 illustrates a case in "determination results related to the present condition of the lungs" are generated as an example of the first determination information. FIG. 5 is a diagram illustrating an example of the first determination information generated from the target image representing a knee joint of the subject. FIG. 5 illustrates a case (example 1) in which "determination results related to the present condition of the knee j oint" are generated as the first determination information, and a case (example 2) in which "determination results related to a future condition of the knee j oint" are generated as the first determination information.

The first generator 211 may include a determination information generation model that can generate the first determination information indicating the condition of the target site of the subject, using the target image of the subject.

The determination information generation model may be a trained first neural network, which is trained by using patient information related to a plurality of patients each having a disorder in the target site as teaching data. The patient information may include the diagnostic information indicating the diagnostic results related to the condition of the target site of each patient and the medical image. The patient information may be further associated with information indicating a time when the medical image is captured. The trained first neural network may be used as the determination information generation model that can output the first determination information from the target image of the subject.

Here, the disorder may be at least one selected from the group consisting of a cancer, a cardiac disease, pneumonia, pulmonary emphysema, cerebral infarction, dementia, osteoarthritis, spondylosis, a bone fracture, and osteoporosis. The osteoarthritis may be hip osteoarthritis or knee osteoarthritis. The bone fracture may be a vertebral compression fracture or a proximal femoral fracture. Here, the first determination information may be a degree of progress or a degree of severity of a disorder, and when the disorder is a cancer, a cardiac disease, and the like, the first determination information may include stage classification indicating a degree of progress or a degree of severity of the disorder. Alternatively, when the disorder is osteoarthritis, spondylosis, and the like, the first determination information may include Kellgren-Lawrence (K-L) classification indicating a degree of progress or a degree of severity of the disorder. Alternatively, when the disorder is a proximal femoral fracture, the first determination information may include a Garden stage indicating a degree of progress or a degree of severity. Here, the first determination information may be a numerical value used to determine whether a disorder is present. For example, when the disorder is osteoporosis, the first determination information may be a value indicating bone density or the like.

In response to the target image being input to an input layer, the first generator 211 performs computation based on the determination information generation model, and outputs the first determination information from an output layer. As an example, the first generator 211 may extract a predetermined feature value from the target image and use the predetermined feature value as input data. In extraction of the feature value, publicly known algorithms as listed below may be applied.
- Convolutional neural network (CNN)
- Autoencoder (auto encoder)
- Recurrent neural network (RNN)
- Long short-term memory (LSTM).

For example, in generation of the determination information generation model that outputs the first determination information related to a disorder in lungs, the following training data and teaching data (ground truth label) are used. As an example, by performing machine learning using backpropagation with application of a CNN on the determination information generation model, the determination information generation model can output accurate determination information related to a disorder in lungs regarding an X-ray image of lungs of any subject.
- Training data: X-ray images of lungs of a plurality of patients.
- Teaching data: Presence or absence of a disorder in the lungs represented in the X-ray image of the lungs of each patient, or a name of the disorder (no problem, a lung cancer, pneumonia, or the like).

For example, in generation of the determination information generation model that outputs the determination information related to a disorder in a knee, the following training data and teaching data (ground truth label) are used. As an example, by performing machine learning using backpropagation with application of a CNN on the determination information generation model, the determination information generation model can output accurate determination information related to a disorder in a knee regarding an X-ray image of a knee of any subject.
- Training data: X-ray images of knees of a plurality of patients.
- Teaching data: Presence or absence of a disorder in the knee represented in the X-ray image of each patient, or a name of the disorder (no problem, osteoarthritis, Kellgren-Laurence classification, or the like).

### Third Generator 212

The third generator 212 generates the region-of-interest information indicating a position of the region of interest, which is a region of interest in the process in which the first determination information is generated by the first generator 211.

Regarding the region-of-interest information, information indicating a position of a partial region of the target image to which a certain layer of the first neural network intensely responded in the process of outputting the first determination information may be output as the region-of-interest information.

The third generator 212 extracts a feature map for the entire target image at a stage when the target image is input to an input layer of the first neural network and processing up to a convolutional layer is executed. The third generator 212 performs segmentation for the target image. Here, segmentation is processing of evaluating, for each pixel of the target image, how the pixel affects the first determination information, and then outputting evaluation results.

In an example, the third generator 212 may identify a position crucial for the first determination information, based on magnitude of a change of output caused when a change of a gradient is added to a certain position of the feature map. In this case, a position that significantly affects the first determination information also has a significant change of the gradient, and a position that does not significantly affect the first determination information also has a not significant change of the gradient.

The third generator 212 outputs, as the region of interest, a region of the target image in the feature map corresponding to the position that significantly affects the first determination information and generates the region-of-interest information indicating the region of interest.

To detect the region of interest, any publicly known algorithm as listed below may be applied.
- Class activation mapping (CAM)
- Gradient-based class activation mapping (Grad-CAM)
- Attention branch network
- Attention guided CNN
- Residual attention network

The first determination information generated by the first generator 211 and the region-of-interest information generated by the third generator 212 are transmitted to the information processing apparatus 1 via the communicator 23, together with the instruction ID.

### Feature Information Generation Apparatus 3

The feature information generation apparatus 3 includes a controller 31 that comprehensively controls each part of the feature information generation apparatus 3, a storage 32 that stores various pieces of data used by the controller 31, and a communicator 33 that performs communication of various pieces of data.

The controller 31 includes a second generator 311 that generates the feature information based on the target image.

### Second Generator 311

The second generator 311 generates the feature information from the target image. The second generator 311 may include a feature generation model that can generate the feature information related to a condition of a body of a subject from the target image of the subject.

The determination information generation model may be a trained second neural network, which is trained by using patient images representing the target site of each of the plurality of patients having a disorder in the target site as teaching data. With the patient image, identification information including a name and an annotation of each feature detected from the patient image and position information indicating a position of each feature in the patient image may be associated. The trained second neural network may be used as a feature information generation model that can output the feature information from the target image of the subject.

Here, the disorder may be at least one selected from the group consisting of a cancer, a cardiac disease, pneumonia, pulmonary emphysema, cerebral infarction, dementia, osteoarthritis, spondylosis, a bone fracture, and osteoporosis.

In response to the target image being input to an input layer, the second generator 311 performs computation based on the feature information generation model and outputs the feature information from an output layer. As an example, the second generator 311 may be configured to extract a predetermined feature value from the target image and use the predetermined feature value as input data. In extraction of the feature value, publicly known algorithms as listed below may be applied.
- Region-based convolutional neural network (R-CNN)
- You only look once (YOLO)
- Single shot detector (SDD).

The feature information generated by the second generator 311 is transmitted to the information processing apparatus 1 via the communicator 33, together with the instruction ID.

The determination system 100 including the above configuration acquires the first determination information, the region-of-interest information, and the feature information generated from the same target image and causes the display apparatus 5 to display these pieces of information. Thus, the determination system 100 can present the user (for example, a medical staff) with the region-of-interest information and the feature information to assist understanding of the first determination information as well as the first determination information. Consequently, the user can correctly understand the first determination information generated from the target image.

For example, in generation of the feature information generation model that outputs the feature information related to an X-ray image of lungs, X-ray images of lungs of a plurality of patients may be used as the training data, and features (ground glass opacity, calcified focus opacity, and the like) of lungs represented in an X-ray image of lungs of each patient and their positions (annotation information) may be used as the teaching data. As an example, by performing machine learning using backpropagation with application of an R-CNN on the feature information generation model, the feature information generation model can output an accurate feature and position related to the feature information of lungs regarding an X-ray image of lungs of any subject.

For example, in generation of the feature information generation model that outputs the feature information related to an X-ray image of a knee, X-ray images of knees of a plurality of patients may be used as the training data, and features (a part corresponding to joint space narrowing, a part corresponding to osteosclerosis, and the like) of a knee represented in an X-ray image of a knee of each patient and their positions (annotation information) may be used as the teaching data. As an example, by performing machine learning using backpropagation with application of an R-CNN on the feature information generation model, the feature information generation model can output an accurate feature and position related to the feature information of a knee regarding an X-ray image of a knee of any subject.

In generation of the feature information, the feature information generation model is applied, which is different from the determination information generation model for outputting the first determination information. Thus, the feature information may include information having high relevance to the first determination information and may simultaneously include information having low relevance to the first determination information and information different from the condition of the target site. The determination system 100 presents the user with the feature information, in addition to the first determination information and the region-of-interest information generated from the target image. Thus, the determination system 100 can inform the user of not only the condition of the target site of the subject but also presence of a feature related to the condition of the body of the subject. Consequently, the user can determine treatment and intervention to be provided to the subject, with an understanding of the condition of the target site of the subject and the condition of the body, not limited to the target site, of the subject.

### Processing Executed by Determination System 100

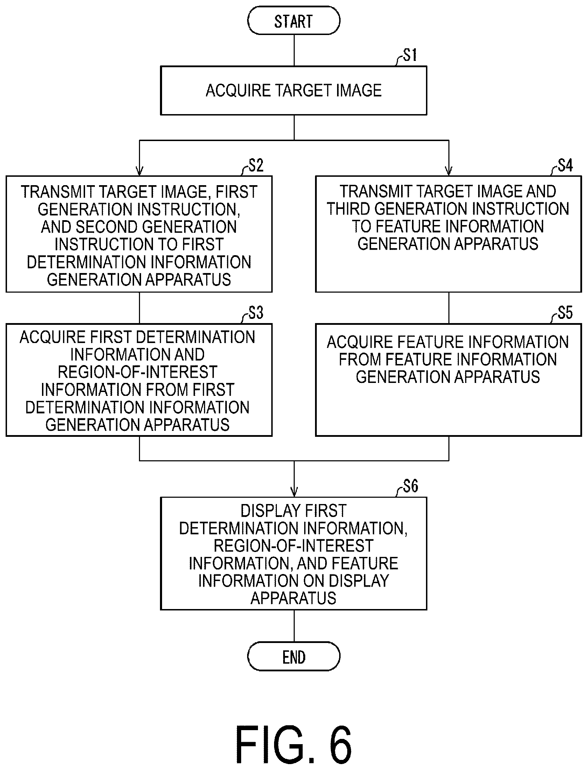

Processing executed by the determination system 100 will be described with reference to FIG. 6. FIG. 6 is a flowchart illustrating an example of a flow of processing executed by the information processing apparatus 1 of the determination system 100.

First, the information processing apparatus 1 acquires the target image from the target image management apparatus 4 or the display apparatus 5 (Step S 1: target image acquisition step).

Subsequently, the information processing apparatus 1 transmits the target image, the first generation instruction, and the second generation instruction to the first determination information generation apparatus 2 (Step S2). In response to the first generation instruction and the second generation instruction, the first determination information generation apparatus 2 generates the first determination information and the region-of-interest information from the target image (a first generation step and a third generation step).

The information processing apparatus 1 acquires the first determination information and the region-of-interest information from the first determination information generation apparatus 2 (Step S3).

On the other hand, the information processing apparatus 1 transmits the target image and the third generation instruction to the feature information generation apparatus 3 (Step S4). In response to the third generation instruction, the feature information generation apparatus 3 generates the feature information from the target image (second generation step).

The information processing apparatus 1 acquires the feature information from the feature information generation apparatus 3 (Step S5).

The information processing apparatus 1 transmits the first determination information and the region-of-interest information acquired in Step S3 and the feature information acquired in Step S5 to the display apparatus 5 and causes the display apparatus 5 to display the pieces of information (Step S6: display control step). The processes of Steps S2 to S3 may be executed either before or after the processes of Steps S4 to S5.

### Display Screen

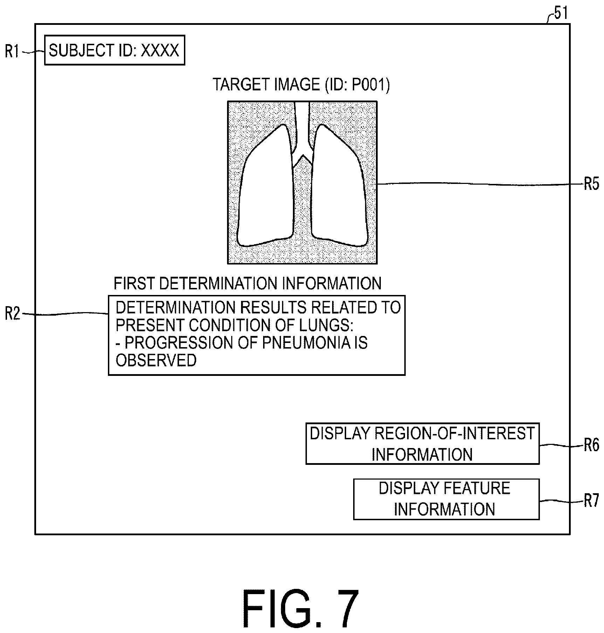

Examples of a display screen displayed on the display 51 of the display apparatus 5 will be described with reference to FIGs. 7 to 12. FIGs. 7 to 12 are each a diagram illustrating an example of a display screen. Here, description will be given by taking an example of a case in which an X-ray image of lungs or a knee joint of a subject is used as the target image.

FIGs. 7 to 12 illustrate examples of a display screen displaying each of the first determination information, the region-of-interest information, and the feature information.

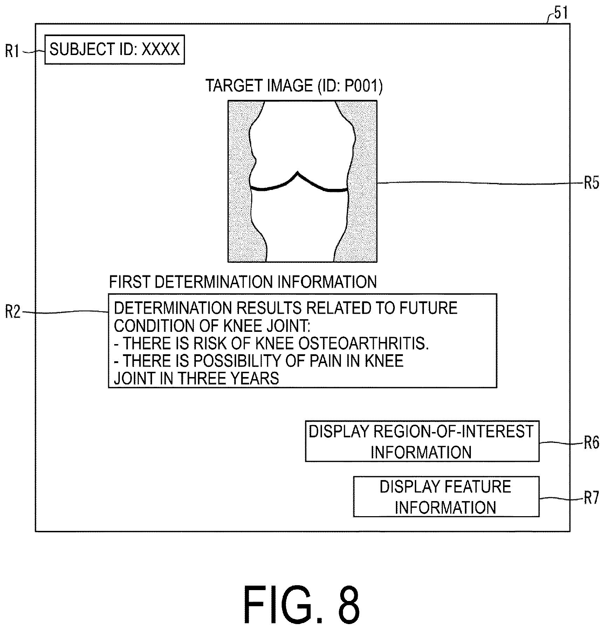

The display screens illustrated in FIGs. 7 and 8 each include a region R1 for displaying the subject ID, a region R2 for displaying the first determination information, and a region R5 for displaying the target image used for generation of the first determination information and its image ID. In the example illustrated in FIG. 7, the region R2 displays the first determination information "Progression of pneumonia is observed". In the example illustrated in FIG. 8, the region R2 displays the first determination information "There is a risk of knee osteoarthritis. There is a possibility of pain in the knee joint in three years".

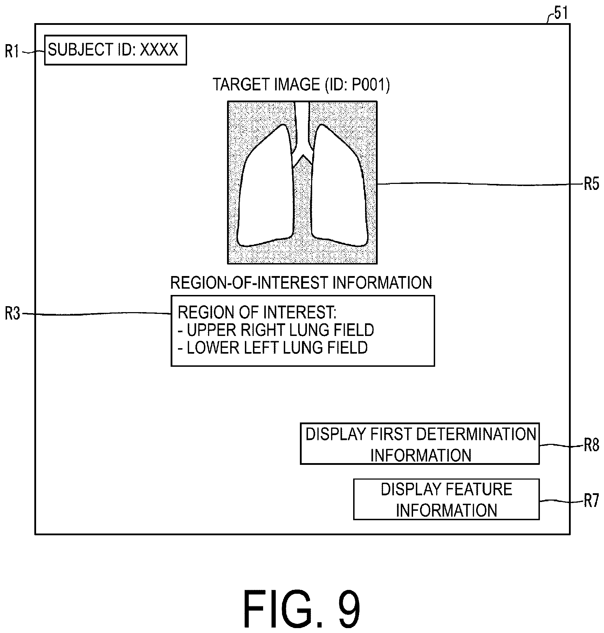

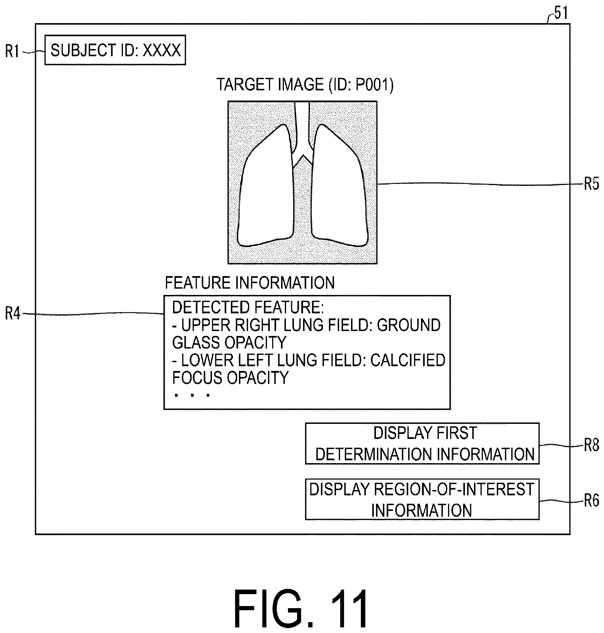

The display screens illustrated in FIGs. 7 and 8 each include a region R6 for receiving an instruction for transitioning to a display screen for displaying the region-of-interest information, and a region R7 for receiving an instruction for transitioning to a display screen for displaying the feature information. For example, when the user selects the region R6 illustrated in FIG. 7, the display screen transitions to the display screen illustrated in FIG. 9. On the other hand, when the user selects the region R7 illustrated in FIG. 7, the display screen transitions to the display screen illustrated in FIG. 11.

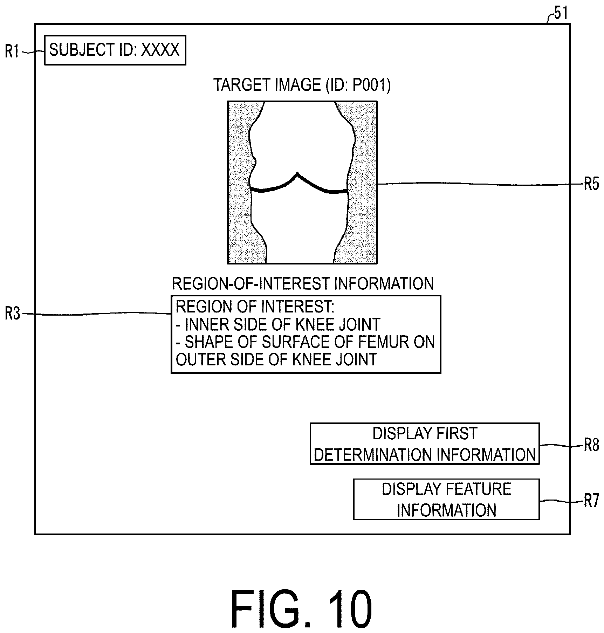

The display screens illustrated in FIGs. 9 and 10 each include a region R1 for displaying the subject ID, a region R3 for displaying the region-of-interest information, and a region R5 for displaying the target image and its image ID. In the example illustrated in FIG. 9, the region R3 displays the region-of-interest information "upper right lung field" and "lower left lung field". In the example illustrated in FIG. 10, the region R3 displays the region-of-interest information "inner side of the knee j oint" and "shape of a surface of the femur on an outer side of the knee joint".

The display screens illustrated in FIGs. 9 and 10 each include a region R8 for receiving an instruction for transitioning to a display screen for displaying the first determination information, and a region R7 for receiving an instruction for transitioning to a display screen for displaying the feature information. For example, when the user selects the region R8 illustrated in FIG. 9, the display screen transitions to the display screen illustrated in FIG. 7. On the other hand, when the user selects the region R7 illustrated in FIG. 10, the display screen transitions to the display screen illustrated in FIG. 11.

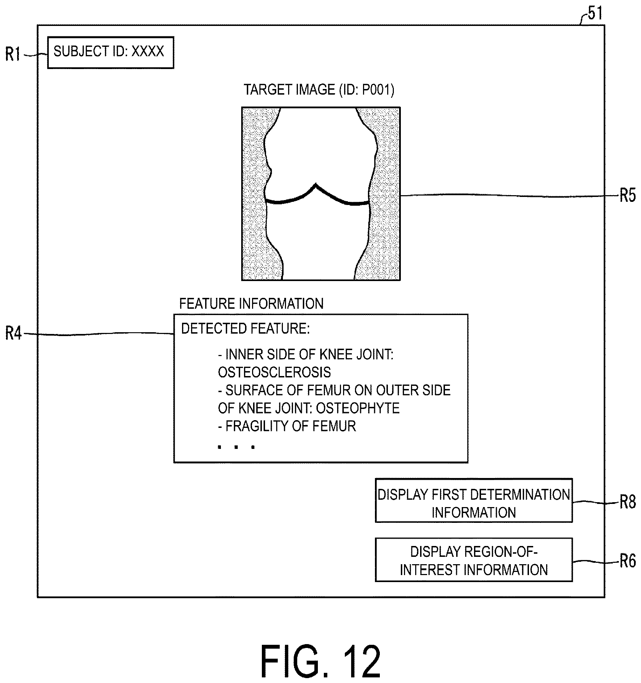

The display screens illustrated in FIGs. 11 and 12 each include a region R1 for displaying the subject ID, a region R4 for displaying the feature information, and a region R5 for displaying the target image and its image ID. In the example illustrated in FIG. 11, the region R4 displays the feature information "upper right lung field: ground glass opacity" and "lower left lung field: calcified focus opacity", for example. In the example illustrated in FIG. 12, the region R4 displays the feature information "inner side of the knee joint: osteosclerosis", "surface of the femur on an outer side of the knee joint: osteophyte", and "fragility of the femur", for example.

The display screens illustrated in FIGs. 11 and 12 each include a region R8 for receiving an instruction for transitioning to a display screen for displaying the first determination information, and a region R6 for receiving an instruction for transitioning to a display screen for displaying the region-of-interest information. For example, when the user selects the region R8 illustrated in FIG. 11, the display screen transitions to the display screen illustrated in FIG. 7. For example, when the user selects the region R6 illustrated in FIG. 11, the display screen transitions to the display screen illustrated in FIG. 9.

### First Variation

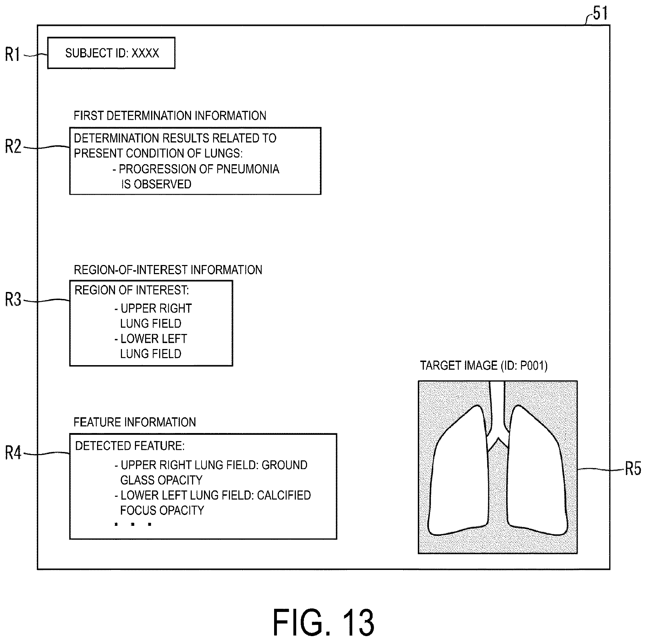

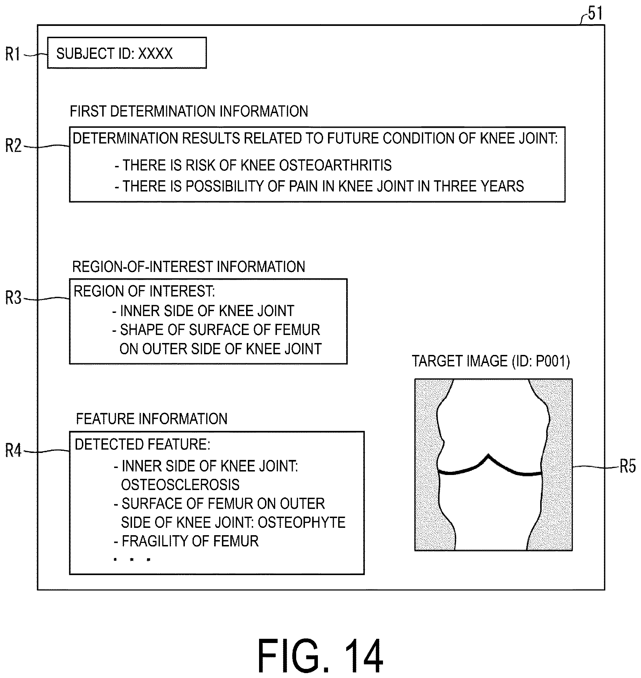

As illustrated in FIGs. 7, 8 to 11, and 12, each of the first determination information, the region-of-interest information, and the feature information may be displayed on the display 51, but this is not restrictive. For example, the first determination information, the region-of-interest information, and the feature information may be collectively displayed on the display 51. Examples of a display screen simultaneously displaying the first determination information, the region-of-interest information, and the feature information will be described with reference to FIGs. 13 and 14. FIGs. 13 and 14 are each a diagram illustrating an example of a display screen.

The display screens illustrated in FIGs. 13 and 14 each include a region R1 for displaying the subject ID, a region R2 for displaying the first determination information, a region R3 for displaying the region-of-interest information, a region R4 for displaying the feature information, and a region R5 for displaying the target image used for generation of the first determination information and its image ID.

In the example illustrated in FIG. 13, the region R2 displays the first determination information "Progression of pneumonia is observed", and the region R3 displays the region-of-interest information "upper right lung field" and "lower left lung field". The region R4 displays the feature information "upper right lung field: ground glass opacity" and "lower left lung field: calcified focus opacity". In the example illustrated in FIG. 14, the region R2 displays the first determination information "There is a risk of knee osteoarthritis" and "There is a possibility of pain in the knee joint in three years". The region R3 displays the region-of-interest information "inner side of the knee j oint" and "shape of a surface of the femur on an outer side of the knee joint". The region R4 displays the feature information "inner side of the knee j oint: osteosclerosis", "surface of the femur on an outer side of the knee joint: osteophyte", and "fragility of the femur", for example.

By collectively presenting the user with the first determination information and the region-of-interest information and the feature information related to the first determination information, which are generated from the same target image of the subject, the user need not view a plurality of screens. According to the configuration, convenience of the determination system 100 can be enhanced.

The examples illustrated in FIGs. 13 and 14 illustrate examples in which the first determination information, the region-of-interest information, and the feature information are simultaneously displayed on one display 51, but this is not restrictive. For example, the first determination information, the region-of-interest information, and the feature information may be simultaneously displayed separately on a plurality of displays 51 designated by the user.

### Second Embodiment

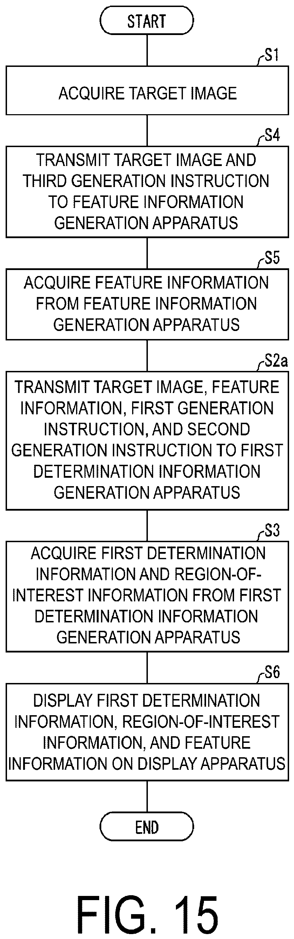

The first generator 211 may include a configuration of taking the feature information generated by the feature information generation apparatus 3 into consideration when generating the first determination information from the target image. A flow of processing performed by the determination system 100 including the configuration will be described with reference to FIG. 15. FIG. 15 is a flowchart illustrating another example of a flow of processing executed by the determination system 100. For the sake of convenience of description, the same processes as the processes described with reference to FIG. 6 are denoted by the same reference signs.

First, the information processing apparatus 1 acquires the target image from the target image management apparatus 4 or the display apparatus 5 (Step S 1: target image acquisition step).

Subsequently, the information processing apparatus 1 transmits the target image and the third generation instruction to the feature information generation apparatus 3 (Step S4). In response to the third generation instruction, the feature information generation apparatus 3 generates the feature information from the target image (second generation step).

The information processing apparatus 1 acquires the feature information from the feature information generation apparatus 3 (Step S5).

Subsequently, the information processing apparatus 1 transmits the target image, the feature information, the first generation instruction, and the second generation instruction to the first determination information generation apparatus 2 (Step S2a). In response to the first generation instruction and the second generation instruction, the first determination information generation apparatus 2 generates the first determination information and the region-of-interest information from the target image and the feature information (a first generation step and a third generation step).

The information processing apparatus 1 acquires the first determination information and the region-of-interest information from the first determination information generation apparatus 2 (Step S3).

The information processing apparatus 1 transmits the feature information acquired in Step S5 and the first determination information and the region-of-interest information acquired in Step S3 to the display apparatus 5 and causes the display apparatus 5 to display the pieces of information (Step S6).

With this configuration, the feature information obtained from the same target image can be utilized for generation of the first determination information. Thus, the determination system 100 can generate and output the first determination information having higher reliability.

### Third Embodiment

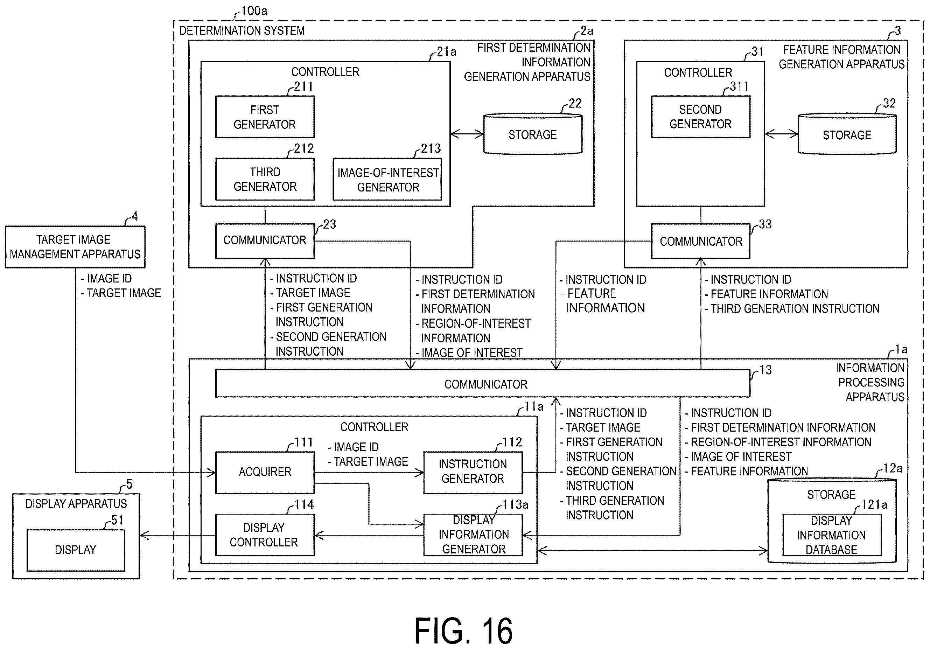

The region-of-interest information may be information indicating a position of a region of interest in the process in which the first determination information is generated from the target image. Thus, an image indicating the region-of-interest information may be generated. Here, a determination system 100a including a first determination information generation apparatus 2a that generates an image of interest indicating the region-of-interest information will be described with reference to FIG. 16. FIG. 16 is a block diagram illustrating an example of a configuration of the determination system 100a. For the sake of convenience of description, members having the same functions as those of the members described in the above-described embodiments are denoted by the same reference signs, and description thereof is not repeated.

The determination system 100a includes an information processing apparatus 1a, a first determination information generation apparatus 2a, and a feature information generation apparatus 3.

The first determination information generation apparatus 2a includes a controller 21a that comprehensively controls each part of the first determination information generation apparatus 2a, a storage 22 that stores various pieces of data used by the controller 21a, and a communicator 23 that performs communication of various pieces of data.

The controller 21a includes a first generator 211 that generates the first determination information based on the target image, a third generator 212 that generates the region-of-interest information, and an image-of-interest generator 213.

The image-of-interest generator 213 generates the image of interest, in response to the second generation instruction. The image-of-interest generator 213 may generate the image of interest, in which the region of interest indicated by the region-of-interest information generated by the third generator 212 is superimposed on the target image.

The first determination information generated by the first generator 211, the region-of-interest information generated by the third generator 212, and the image of interest generated by the image-of-interest generator 213 are transmitted to the information processing apparatus 1a via the communicator 23, together with the instruction ID.

A display information generator 113a of the information processing apparatus 1a acquires, for each target image, (1) the first determination information, the region-of-interest information, and the image of interest generated by the first determination information generation apparatus 2a and (2) the feature information generated by the feature information generation apparatus 3. The display information generator 113a refers to the instruction ID and the like associated with each piece of acquired information, associates the first determination information, the region-of-interest information, and the image of interest and the feature information, and generates display information. The display information generator 113a may store the generated display information in a display information database 121a of a storage 12a, together with the instruction ID.

### Display Information Database 121a

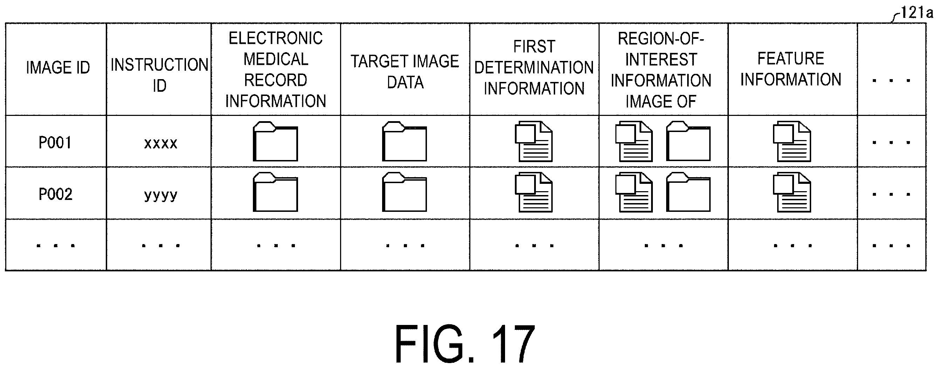

Here, the display information database 121a will be described with reference to FIG. 17. FIG. 17 is a diagram illustrating an example of a data structure of the display information database 121a.

The display information database 121a records the following pieces of information, which are associated with each other.
- The instruction ID.
- The first determination information, the region-of-interest information, and the image of interest generated by the first determination information generation apparatus 2 in response to the first generation instruction and the second generation instruction assigned the instruction ID.
- The feature information generated by the feature information generation apparatus 3 in response to the third generation instruction assigned the instruction ID.

Referring back to FIG. 16, the display controller 114 of the information processing apparatus 1a causes the display 51 of the display apparatus 5 to perform display, using each piece of information associated for each instruction ID from the display information database 121a.

According to the configuration, the determination system 100a can clearly show the user which region of the target image is the region of interest. Thus, the determination system 100a can enhance convenience of the region-of-interest information.

### Fourth Embodiment

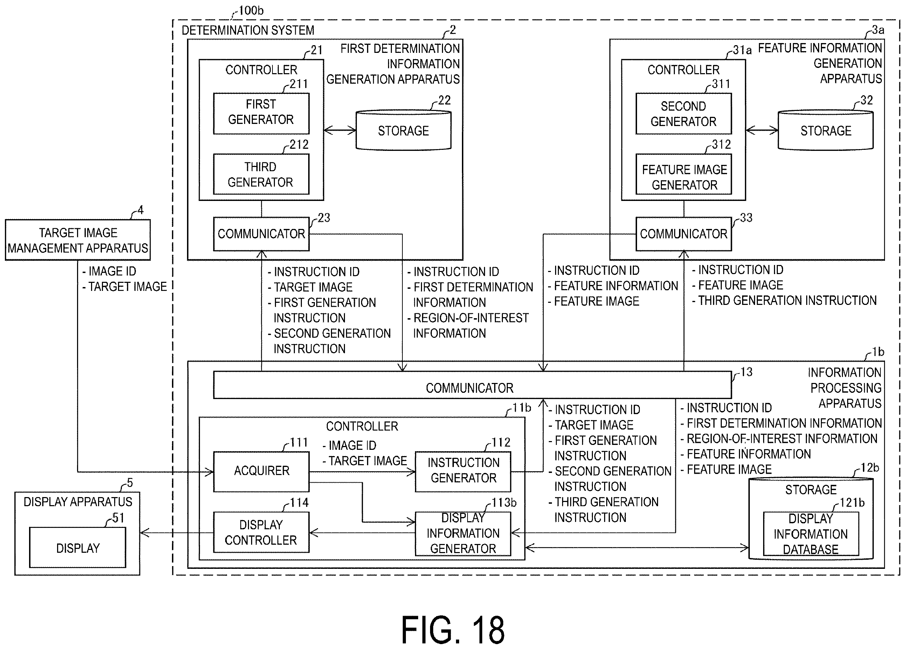

The feature information is information indicating a position of the feature detected from the target image. Thus, a first feature image, in which the feature information is superimposed on the target image, may be generated. Here, a determination system 100b including a feature information generation apparatus 3a that generates a feature image, in which the feature information is superimposed on the target image, will be described with reference to FIG. 18. FIG. 18 is a block diagram illustrating an example of a configuration of the determination system 100b. For the sake of convenience of description, members having the same functions as those of the members described in the above-described embodiments are denoted by the same reference signs, and description thereof is not repeated.

The determination system 100b includes an information processing apparatus 1b, a first determination information generation apparatus 2, and a feature information generation apparatus 3 a.

The feature information generation apparatus 3a includes a controller 31a that comprehensively controls each part of the feature information generation apparatus 3a, a storage 32 that stores various pieces of data used by the controller 31a, and a communicator 33 that performs communication of various pieces of data.

The controller 31a includes a second generator 311 that generates the feature information based on the target image, and a feature image generator 312.

The feature image generator 312 generates the first feature image, in response to the third generation instruction. The feature image generator 312 may generate the first feature image in which the feature information generated by the second generator 311 is superimposed on the target image.

The feature information generated by the second generator 311 and the feature image generated by the feature image generator 312 are transmitted to the information processing apparatus 1b via the communicator 33, together with the instruction ID.

A display information generator 113b of the information processing apparatus 1b acquires, for each target image, (1) the first determination information and the region-of-interest information generated by the first determination information generation apparatus 2 and (2) the feature information and the feature image generated by the feature information generation apparatus 3a. The display information generator 113b refers to the instruction ID and the like associated with each piece of acquired information, associates the first determination information and the region-of-interest information and the feature information and the feature image, and generates display information. The display information generator 113b may store the generated display information in a display information database 121b of a storage 12b, together with the instruction ID.

The display information database 121b records the following pieces of information, which are associated with each other.
- The instruction ID.
- The first determination information and the region-of-interest information generated by the first determination information generation apparatus 2 in response to the first generation instruction and the second generation instruction assigned the instruction ID.
- The feature information and the feature image generated by the feature information generation apparatus 3 in response to the third generation instruction assigned the instruction ID.

The display controller 114 of the information processing apparatus 1b causes the display 51 of the display apparatus 5 to perform display, using each piece of information associated for each instruction ID from the display information database 121b.

According to the configuration, the determination system 100b can clearly show the user in which region of the target image the feature is detected. Thus, the determination system 100b can enhance convenience of the feature information.

### Fifth Embodiment

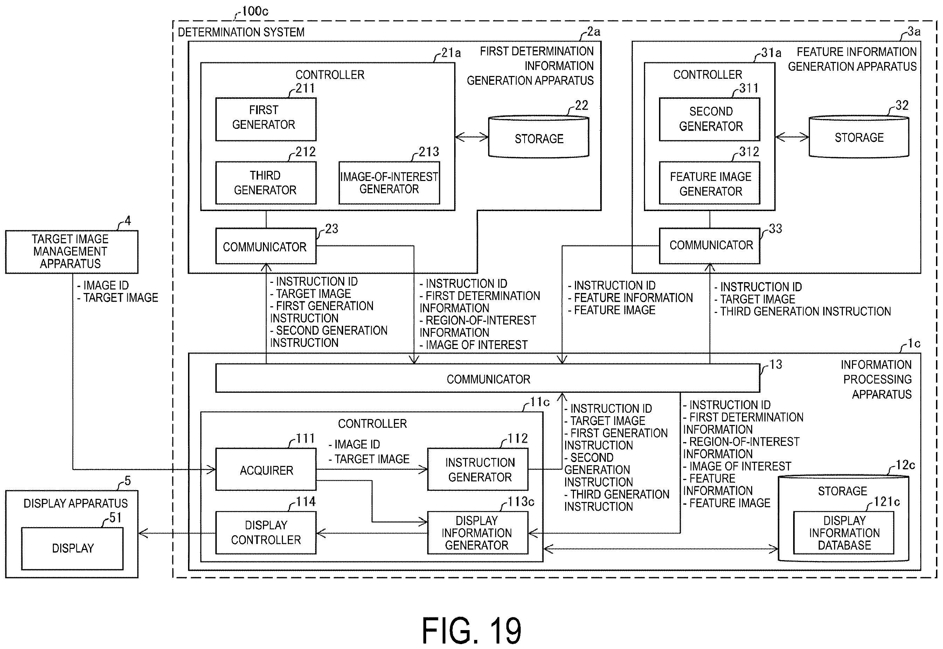

The image of interest and the feature image may be generated. Here, a determination system 100c including an image-of-interest generator 213 that generates the image of interest in which the region-of-interest information is superimposed on the target image and a feature information generation apparatus 3a that generates the first feature image in which the feature information is superimposed on the target image will be described with reference to FIG. 19. FIG. 19 is a block diagram illustrating an example of a configuration of the determination system 100c. For the sake of convenience of description, members having the same functions as those of the members described in the above-described embodiments are denoted by the same reference signs, and description thereof is not repeated.

The determination system 100c includes an information processing apparatus 1c, a first determination information generation apparatus 2a, and a feature information generation apparatus 3 a.

A display information generator 113c of the information processing apparatus 1c acquires for each target image, (1) the first determination information, the region-of-interest information, and the image of interest generated by the first determination information generation apparatus 2a and (2) the feature information and the feature image generated by the feature information generation apparatus 3a. The display information generator 113c refers to the instruction ID and the like associated with each piece of acquired information, associates the first determination information, the region-of-interest information, and the image of interest and the feature information and the feature image, and generates display information. The display information generator 113c may store the generated display information in a display information database 121c of a storage 12c, together with the instruction ID.

The display controller 114 of the information processing apparatus 1c causes the display 51 of the display apparatus 5 to perform display, using each piece of information associated for each instruction ID from the display information database 121c.

### Display Information Database 121c

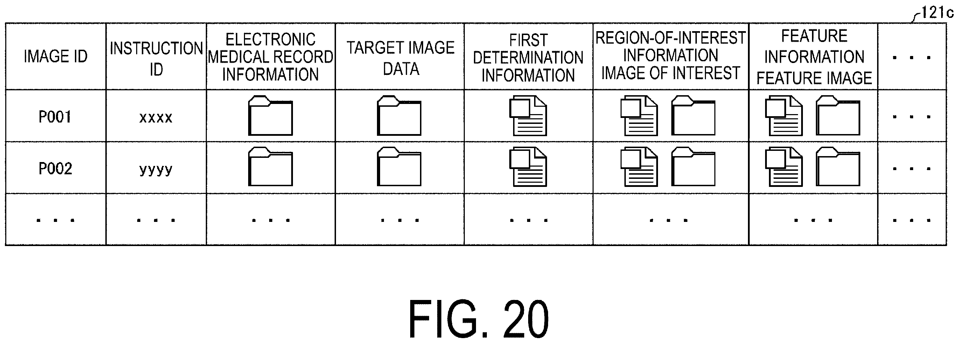

Here, the display information database 121c will be described with reference to FIG. 20. FIG. 20 is a diagram illustrating an example of a data structure of the display information database 121c.

The display information database 121c records the following pieces of information, which are associated with each other.
- The instruction ID.
- The first determination information, the region-of-interest information, and the image of interest generated by the first determination information generation apparatus 2a in response to the first generation instruction and the second generation instruction assigned the instruction ID.
- The feature information and the feature image generated by the feature information generation apparatus 3a in response to the third generation instruction assigned the instruction ID.

Referring back to FIG. 19, the display controller 114 of the information processing apparatus 1c causes the display 51 of the display apparatus 5 to perform display, using each piece of information associated for each instruction ID from the display information database 121c.

According to the configuration, the determination system 100c can clearly show the user which region of the target image is the region of interest and from where the feature is detected. Thus, the determination system 100c can enhance convenience of the region-of-interest information and the feature information.

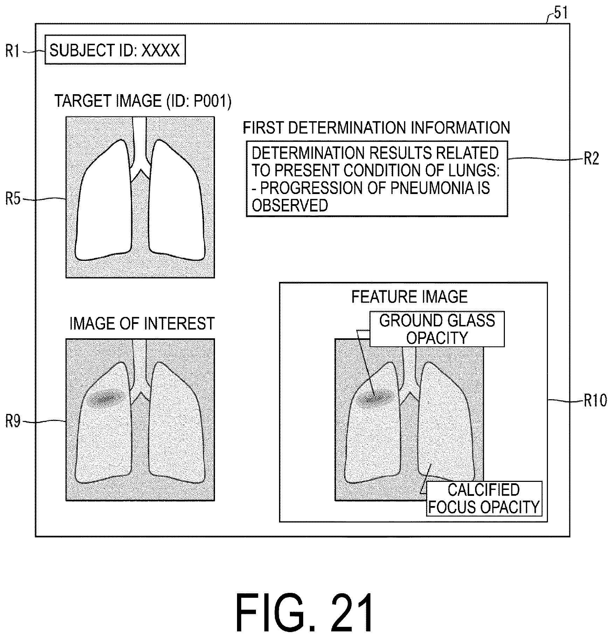

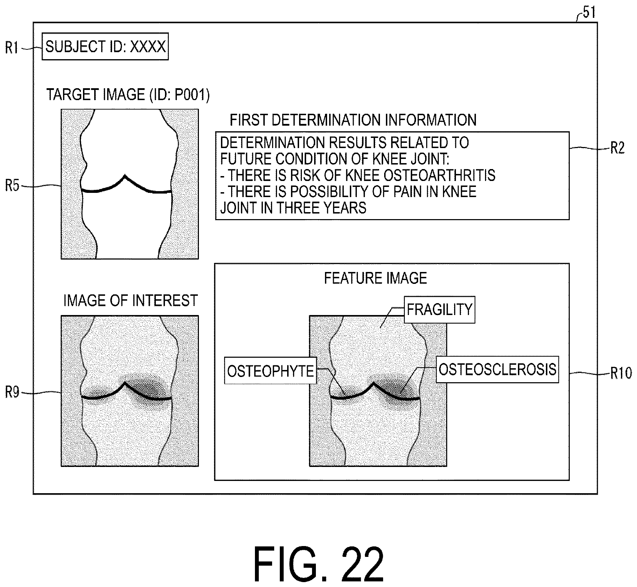

The information processing apparatus 1c may include a function of the feature image generator 312. In this case, the information processing apparatus 1c can generate a second feature image from the region-of-interest information or the image of interest generated by the first determination information generation apparatus 2a and the feature information acquired from the feature information generation apparatus 3a. The second feature image is an image in which the feature information and the region-of-interest information are superimposed on the target image. The second feature image may be an image in which the feature information is superimposed on the image of interest (see FIGs. 21 and 22).

### Display Screen

Examples of a display screen displayed on the display 51 of the display apparatus 5 will be described with reference to FIGs. 21 and 22. FIGs. 21 and 22 are each a diagram illustrating an example of a display screen. In FIG. 21, description will be given by taking an example of a case in which an X-ray image of lungs of a subject is used as the target image, and the image of interest is displayed instead of the region-of-interest information and the second feature image is displayed instead of the feature information. In FIG. 22, an X-ray image of a knee joint of a subject is used as the target image.

The display screens illustrated in FIGs. 21 and 22 each include a region R1 for displaying the subject ID, a region R2 for displaying the first determination information, and a region R5 for displaying the target image used for generation of the first determination information and its image ID.

The display screens illustrated in FIGs. 21 and 22 each include a region R9 for displaying the image of interest, and a region R10 for displaying the second feature image. Instead of the second feature image, the first feature image may be displayed in the region R10.

As illustrated in the region R9 of FIGs. 21 and 22, in an example, in the image of interest, the region of interest may be represented as a heat map on the target image. In contrast, as illustrated in the region R10, in an example, the feature image may be an image in which positions of detected features and names of their respective features are displayed on the target image or the image of interest.

In this manner, the determination system 100c may cause the display apparatus to display the image of interest and the second feature image alongside each other. This allows the user to view and compare the image of interest and the second feature image with each other, and comprehensively perceive the first determination information, the region-of-interest information, and the feature information generated from the same target image. Thus, the determination system 100c can have the user understand the first determination information and can effectively prompt the user to utilize the first determination information.

### Sixth Embodiment

Another embodiment of the present disclosure will be described below. For the sake of convenience of description, members having the same functions as those of the members described in the above-described embodiments are denoted by the same reference signs, and description thereof is not repeated.

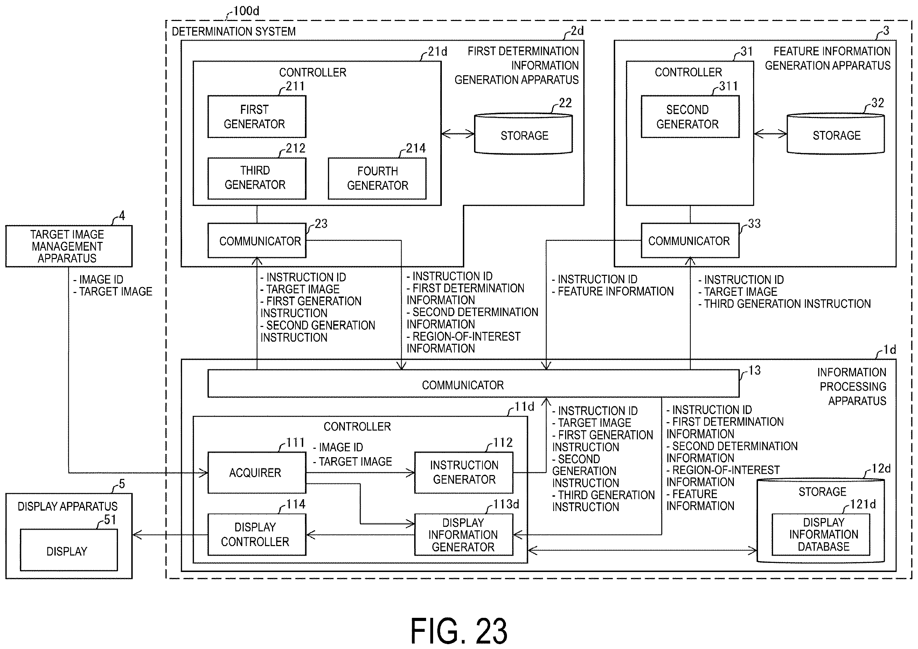

Second determination information indicating a method of medical intervention for the subject and an effect from the medical intervention may be generated from the first determination information. A configuration of the determination system 100d that can generate the second determination information will be described with reference to FIG. 23. FIG. 23 is a functional block diagram illustrating an example of a configuration of the determination system 100d. For the sake of convenience of description, members having the same functions as those of the members described in the above-described embodiments are denoted by the same reference signs, and description thereof is not repeated.

The determination system 100d includes an information processing apparatus 1d, a first determination information generation apparatus 2d, and a feature information generation apparatus 3.

The first determination information generation apparatus 2d includes a controller 21d that comprehensively controls each part of the first determination information generation apparatus 2d, a storage 22 that stores various pieces of data used by the controller 21d, and a communicator 23 that performs communication of various pieces of data.

### Fourth Generator 214

A fourth generator 214 generates the second determination information indicating a method of medical intervention for the subject and an effect from the medical intervention from the first determination information generated by the first generator 211. In an example, the fourth generator 214 may generate the second determination information, in response to the first generation instruction.

The fourth generator 214 generates the second determination information related to an effect obtained when a medical intervention is provided to the subject from the first determination information generated by the first generator 211.

The fourth generator 214 may include an intervention effect determination model that receives input of the first determination information of the subject, and outputs the second determination information indicating a method of intervention for the subject and an effect from the intervention.

The intervention effect determination model may be a trained third neural network, which is trained by using effect information of each of the plurality of patients who have undergone interventions for disorders in the target site as teaching data. The effect information may be information in which the intervention provided for the target site of each patient and intervention effect information indicating an effect from the intervention are associated for each patient. The trained third neural network may be used as the intervention effect determination model that can output the second determination information from the first determination information.

Here, the intervention may include at least one selected from the group consisting of diet therapy, exercise therapy, pharmacotherapy, stimulation therapy, manual therapy, ultrasound therapy, use of a walking aid, use of an orthosis, a correction surgery, an osteotomy, an intra-articular injection, a j oint preservation surgery, an artificial joint replacement surgery, and a spinal instrumentation surgery.

The first determination information generated by the first generator 211, the region-of-interest information generated by the third generator 212, and the second determination information generated by the fourth generator 214 are transmitted to the information processing apparatus 1d via the communicator 23, together with the instruction ID.

A display information generator 113d of the information processing apparatus 1d acquires, for each target image, (1) the first determination information, the region-of-interest information, and the second determination information generated by the first determination information generation apparatus 2d and (2) the feature information generated by the feature information generation apparatus 3. The display information generator 113d refers to the instruction ID and the like associated with each piece of acquired information, associates the first determination information, the region-of-interest information, and the second determination information and the feature information, and generates display information. The display information generator 113d may store the generated display information in a display information database 121d of a storage 12d, together with the instruction ID.

The display controller 114 of the information processing apparatus 1d causes the display 51 of the display apparatus 5 to perform display, using each piece of information associated for each instruction ID from the display information database 121d.

According to the configuration, the determination system 100d can present the user with the second determination information related to an effect obtained when a medical intervention is provided to the subject, in addition to the first determination information related to the condition of the target site of the subject, from the target image of the subject. Thus, the user of the determination system 100d can appropriately judge whether a medical intervention for the subject is necessary or not.

For example, the fourth generator 214 related to an X-ray image of lungs is generated using training with application of backpropagation in LSTM using the following training data and the following teaching data. Such a fourth generator 214 allows for prediction of an accurate intervention effect for lungs regarding an X-ray image of lungs of any subject.
- Training data: Combinations of X-ray images of lungs of a plurality of patients and interventions provided for the patients.
- Teaching data: Information related to a prognostic effect of the lungs represented in each of the X-ray images of the lungs of the plurality of patients (presence or absence of an effect, a change in length of hospital stay, and the like).

For example, the fourth generator 214 related to an X-ray image of a knee is generated using training with application of backpropagation in LSTM using the following training data and the following teaching data. Such a fourth generator 214 allows for prediction of an accurate intervention effect for a knee regarding an X-ray image of a knee of any subject.
- Training data: Combinations of X-ray images of knees of a plurality of patients and interventions provided for the patients.
- Teaching data: Information related to a prognostic effect of the knee represented in each of the X-ray images of the knee of the plurality of patients (a change in pain, improvement in walking ability, and the like).

### Seventh Embodiment

Another embodiment of the present disclosure will be described below. For the sake of convenience of description, members having the same functions as those of the members described in the above-described embodiments are denoted by the same reference signs, and description thereof is not repeated.

### Configuration of Determination System 100

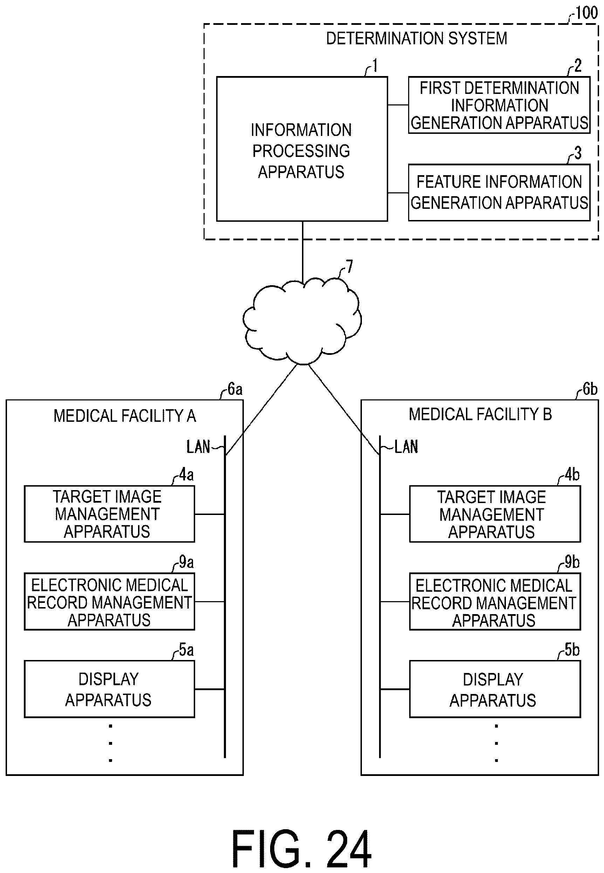

The information processing apparatus 1 illustrated in FIG. 1 may be communicably connected to LANs respectively disposed in a plurality of medical facilities 6 via a communication network 7, instead of a computer installed in the predetermined medical facility 6. FIG. 24 is a block diagram illustrating a configuration example of the determination system 100 according to another aspect of the present disclosure.

In the determination system 100 illustrated in FIG. 24, the information processing apparatus 1 is communicably connected to respective apparatuses of medical facilities 6a and 6b via the communication network 7.

The medical facility 6a includes a display apparatus 5a, a target image management apparatus 4a, and an electronic medical record management apparatus 9a, which are communicably connected to each other. On the other hand, the medical facility 6b includes a display apparatus 5b, a target image management apparatus 4b, and an electronic medical record management apparatus 9b, which are communicably connected to each other.

FIG. 24 illustrates an example in which the LANs of the medical facility 6a and the medical facility 6b are connected to the communication network 7. The information processing apparatus 1 is communicably connected to respective apparatuses of the medical facilities 6a and 6b via the communication network 7 and is not limited to the configuration illustrated in FIG. 24.

In the determination system 100 employing such a configuration, the information processing apparatus 1 can acquire a target image of a subject Pa from the medical facility 6a and acquire a target image of a subject Pb from the medical facility 6b.

In this case, the target image of each subject includes identification information specific to each of the medical facilities 6a and 5b assigned for each medical facility 6 where each subject is examined and identification information specific to each subject assigned for each subject. The identification information specific to each of the medical facilities 6a and 6b may be a facility ID, for example. The identification information specific to each subject may be a patient ID, for example. The information processing apparatus 1 can transmit various pieces of information acquired from the first determination information generation apparatus 2 and the feature information generation apparatus 3 to the display apparatuses 5a and 5b respectively installed in the medical facilities 6a and 6b being transmission sources of the target images.

The information processing apparatus 1 can correctly transmit the first determination information, the region-of-interest information, and the feature information indicating the condition of the target site of the subjects to the display apparatuses 5a and 5b of the medical facilities 6a and 6b where the subjects have undergone the examination, based on these pieces of identification information.

Each of the information processing apparatus 1, the first determination information generation apparatus 2, and the feature information generation apparatus 3 may be connected to the communication network 7. Alternatively, the first determination information generation apparatus 2 and/or the feature information generation apparatus 3 may be disposed in one of the medical facilities 6a and 6b.

In the determination system 100a to 100d, the information processing apparatuses 1a to 1d may be communicably connected to the LANs respectively disposed in the medical facilities 6a and 6b via the communication network 7. Each of the information processing apparatuses 1a to 1d, the first determination information generation apparatuses 2 and 2a, and the feature information generation apparatuses 3 and 3a may be connected to the communication network 7. Alternatively, the first determination information generation apparatuses 2 and 2a and/or the feature information generation apparatuses 3 and 3a may be disposed in one of the medical facilities 6a and 6b.

### Supplementary Note 1

In the embodiments described above, description has been given by taking an example of a configuration in which the acquirer 111 of the information processing apparatuses 1 and 1a to 1d acquires the target image from the target image management apparatus 4, and the target image is transmitted to the first determination information generation apparatuses 2, 2a, and 2d and the feature information generation apparatuses 3 and 3a. However, these are not restrictive.

For example, the first determination information generation apparatuses 2, 2a, and 2d and the feature information generation apparatuses 3 and 3a may acquire the target image from the target image management apparatus 4. In this case, various generation instructions transmitted from the information processing apparatuses 1 and 1a to 1d to the first determination information generation apparatuses 2, 2a, and 2d and the feature information generation apparatuses 3 and 3a may each include the image ID being identification information assigned to the target image.

The first determination information generation apparatuses 2, 2a, and 2d acquire, from the target image management apparatus 4, the target image assigned the image ID included in the first generation instruction and the second generation instruction received from the information processing apparatuses 1 and 1a to 1d.

### Supplementary Note 2

In the embodiments described above, description has been given by taking an example of a configuration in which the first determination information generation apparatuses 2, 2a, and 2d generate the first determination information and the region-of-interest information. However, this is not restrictive.

For example, the information processing apparatuses 1 and 1a to 1d may include the functions of the first generator 211 and the third generator 212 (and the image-of-interest generator 213). The information processing apparatuses 1 and 1a to 1d may include the function of the image-of-interest generator 213 and/or the function of the fourth generator 214.

### Supplementary Note 3

In the embodiments described above, description has been given by taking an example of a configuration in which the feature information generation apparatuses 3 and 3a generate the feature information. However, this is not restrictive.

For example, the information processing apparatuses 1 and 1a to 1d may include the function of the second generator 311. The information processing apparatuses 1 and 1a to 1d may include the function of the feature image generator 312.

### Supplementary Note 4

In FIG. 24, in the embodiments described above of the information processing apparatus 1 and the determination system 100, description has been given by taking an example of a configuration in which the feature information generation apparatuses 3 and 3a generate the feature information. However, this is not restrictive.

For example, the information processing apparatuses 1 and 1a to 1d may include the function of the second generator 311. The information processing apparatuses 1 and 1a to 1d may include the function of the feature image generator 312.

### Example of Software Implementation

Each control block of the determination systems 100 and 100a to 100d (particularly, the controllers 11, 11a to 11d, 21, 21a, 21d, 31, and 31a) may be implemented by a logic circuit (hardware) formed in an integrated circuit (IC chip) or the like, or may be implemented by software.

In the latter case, the determination systems 100 and 100a to 100d include a computer that executes instructions of a program, which is software that implements the functions. The computer includes, for example, one or more processors and a computer-readable recording medium that stores the above program. In the computer, the processor reads the above program from the recording medium and executes the read program to achieve the object of the present disclosure. As the processor, a central processing unit (CPU) can be used, for example. As the recording medium, a "non-transitory tangible medium", for example, a tape, a disk, a card, a semiconductor memory, a programmable logic circuit, or the like can be used in addition to a read only memory (ROM). The computer may further include a random access memory (RAM) for loading the above program. The above program may be supplied to the computer via any transmission medium (communication network, broadcast waves, and the like) that can transmit the program. An aspect of the present disclosure may be implemented in the form of data signals embedded in a carrier wave in which the above program is embodied by electronic transmission.

The invention according to the present disclosure has been described above based on the various drawings and examples. However, the invention according to the present disclosure is not limited to each embodiment described above. That is, the embodiments of the invention according to the present disclosure can be changed in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or modifications based on the present disclosure. Note that these variations or modifications are included within the scope of the present disclosure.

### REFERENCE SIGNS

1, 1a to 1d Information processing apparatus
2, 2a, 2d First determination information generation apparatus
3, 3a Feature information generation apparatus
4, 4a, 4b Target image management apparatus
5, 5a, 5b Display apparatus
6, 6a, 6b Medical facility
7 Communication network
9, 9a, 9b Electronic medical record management apparatus
11, 11a to 11d, 21, 21a, 21d, 31, 31a Controller
12, 12a to 12d, 22, 32 Storage
13, 23, 33 Communicator
51 Display
100, 100a to 100d Determination system
111 Acquirer
112 Instruction generator
113, 113a to 113d Display information generator
114 Display controller
211 First generator
212 Third generator
213 Image-of-interest generator
214 Fourth generator
311 Second generator
312 Feature image generator

## Claims

1. A determination system comprising:
an acquirer configured to acquire a target image representing a target site of a body of a subject;
a first generator configured to generate first determination information indicating a condition of the target site from the target image;
a second generator configured to generate feature information indicating features related to a condition of the body of the subject from the target image used to generate the first determination information; and
a display controller configured to acquire the first determination information and the feature information and cause a display apparatus to display the first determination information and the feature information.

2. The determination system according to claim 1, further comprising
a third generator configured to generate region-of-interest information indicating a region of interest related to the first determination information and being a partial region of the target image,
wherein the display controller acquires the first determination information, the feature information, and the region-of-interest information and cause the display apparatus to display the first determination information, the feature information, and the region-of-interest information.

3. The determination system according to claim 2,
wherein the region of interest comprises a region of interest in a process of generating the first determination information from the target image, and
the region-of-interest information comprises information indicating a position of the region of interest in the target image.

4. The determination system according to any one of claims 1 to 3,
wherein the feature information comprises identification information comprising a name of each of the features detected from the target image and position information indicating a position of each of the features in the target image.

5. The determination system according to any one of claims 2 to 4, further comprising
an image-of-interest generator configured to generate an image of interest where a region of interest indicated by the region-of-interest information indicating the region of interest related to the first determination information and being a partial region of the target image is superimposed on the target image,
wherein the display controller causes the display apparatus to display the image of interest.

6. The determination system according to claim 5, further comprising
a feature image generator configured to generate a first feature image where the feature information is superimposed on the target image and/or a second feature image where the feature information and the region-of-interest information are superimposed on the target image,
wherein the display controller causes the display apparatus to display the first feature image and/or the second feature image.

7. The determination system according to claim 6,
wherein the display controller causes the display apparatus to display the image of interest and the second feature image alongside each other.

8. The determination system according to any one of claims 2 to 7,
wherein the display controller causes the display apparatus to simultaneously display (1) the first determination information, (2) the region-of-interest information indicating the region of interest related to the first determination information and being a partial region of the target image, and (3) the feature information.

9. The determination system according to any one of claims 1 to 8,
wherein the target image comprises a medical image representing the target site of the subj ect.

10. The determination system according to any one of claims 1 to 9,
wherein the first determination information comprises diagnostic information comprising diagnostic results related to whether the subject has a disorder.

11. The determination system according to any one of claims 1 to 10,
wherein the first generator comprises a determination information generation model configured to generate the first determination information indicating a condition of the target site of the subject by using the target image of the subject.

12. The determination system according to claim 11,
wherein the determination information generation model comprises a first neural network trained by using patient information, as teaching data, related to a plurality of patients each having a disorder in a target site, and
the patient information comprises diagnostic information indicating diagnostic results related to a condition of a target site and a medical image of each of the plurality of patients.

13. The determination system according to claim 12,
wherein the patient information is associated with the diagnostic information and the medical image of each of the plurality of patients and information indicating a time when the medical image is captured.

14. The determination system according to any one of claims 1 to 13,
wherein the second generator comprises a feature generation model configured to generate the feature information related to the condition of the body of the subject from the target image of the subject.

15. The determination system according to claim 14,
wherein the feature generation model comprises a second neural network trained by using a patient image, as teaching data, representing the target site of each of the plurality of patients having the disorder in the target site, and
the patient image is associated with identification information comprising a name and an annotation of each of the features detected from the patient image and position information indicating a position of each of the features in the patient image.

16. The determination system according to any one of claims 12, 13, and 15,
wherein the disorder comprises at least one selected from the group consisting of a cancer, a cardiac disease, pneumonia, pulmonary emphysema, cerebral infarction, dementia, osteoarthritis, spondylosis, a bone fracture, and osteoporosis.

17. The determination system according to any one of claims 1 to 16,
wherein the feature comprises at least one selected from the group consisting of an image of a malignant tumor, an image of a benign tumor, an image of a thrombus, an image of angiosclerosis, ground glass opacity, a crazy-paving pattern, a patchy shadow, an image of interstitial change, an image of a pulmonary cyst, an image of pulmonary emphysema, an image of nerve compression, an image of a meniscus injury, an image of a ligament injury, an image of osteosclerosis, an image of a bone fracture, and an image of joint space narrowing.

18. The determination system according to any one of claims 1 to 17,
wherein the target image comprises any one of an X-ray image, a CT image, an MRI image, a PET image, an RI image, a mammographic image, an ultrasonic image, an endoscopic image, and an angiographic image.

19. The determination system according to any one of claims 1 to 18,
wherein the first determination information comprises information used to determine the condition of the target site at a second time after elapse of a predetermined period since a first time when the target image is captured.

20. The determination system according to any one of claims 1 to 19, further comprising
a fourth generator configured to generate, from the first determination information, second determination information indicating a method of medical intervention for the subject and an effect of the medical intervention,
wherein the display controller causes the display apparatus to display the first determination information, the feature information generated from the target image used to generate the first determination information, and the second determination information.

21. The determination system according to claim 20,
wherein the fourth generator comprises an intervention effect determination model configured to receive input of the first determination information of the subject and output the second determination information indicating a method of intervention for the subject and an effect of the intervention.

22. The determination system according to claim 21,
wherein the intervention effect determination model comprises a third neural network trained by using effect information, as teaching data, of each of the plurality of patients who have undergone intervention for a disorder in the target site, and
the effect information comprises information where the intervention provided for the target site of each of the plurality of patients and intervention effect information indicating the effect of the intervention are associated for each of the plurality of patients.

23. A determination system comprising:
an information processing apparatus comprising the acquirer and the display controller according to claim 1;
a first determination information generation apparatus comprising the first generator according to claim 1 and the third generator according to claim 2; and
a feature information generation apparatus comprising the second generator according to claim 1.

24. A method of controlling a determination system, the method comprising:
acquiring a target image representing a target site of a body of a subject;
generating first determination information obtained by determining a condition of the target site from the target image;
generating feature information indicating features related to a condition of the body of the subject from the target image used to generate the first determination information; and
acquiring the first determination information and the feature information and causing a display apparatus to display the first determination information and the feature information.

25. A control program for causing a computer to operate as the determination system according to any one of claims 1 to 23, the control program causing the computer to operate as the acquirer, the first generator, the second generator, and the display controller.

26. A control program for causing a computer to operate as the information processing apparatus according to claim 23, the control program causing the computer to execute:
transmitting the target image or identification information assigned to the target image to the information processing apparatus and the feature information generation apparatus; and
acquiring the first determination information and the feature information generated from the transmitted target image and causing a display apparatus to display the first determination information and the feature information.
